# EUROPÄISCHE PATENTANMELDUNG

(11) **EP 0 867 455 A2**
(43) Veröffentlichungstag der Anmeldung: **30.09.1998**
(21) Anmeldenummer: 98105250.9
(22) Anmeldetag: 23.03.1998
(51) Int. Cl.: C08F 226/06, C08F 226/02, C09D 139/00, C09J 133/00

(54) **Wässrige Copolymerisatdispersionen aus wasserlöslichen Monomeren mit N-Vinylgruppen und hydrophoben Monomeren**

(30) Priorität: 24.03.1997 DE 19712247; 03.11.1997 DE 19748545
(71) Anmelder: BASF AKTIENGESELLSCHAFT, 67056 Ludwigshafen (DE)
(72) Erfinder: Blankenburg, Rainer, 70469 Stuttgart-Feuerbach (DE); Schehlmann, Volker, 67354 Römerberg (DE); Kolter, Karl, 67117 Limburgerhof (DE)
(74) Vertreter: Kinzebach, Werner, Dr.

(57) **Zusammenfassung**

Die vorliegende Erfindung betrifft die Herstellung wässriger Copolymerisatdispersion aus
i) 10 bis 70 Gew.-% wenigstens eines wasserlöslichen, N-Vinylgruppen enthaltenden Monomers,
ii) 30 bis 90 Gew.-% wenigstens eines hydrophoben Monomers B mit einer Wasserlöslichkeit unterhalb 60 g/l bei 25 °C sowie gegebenenfalls
iii) 0 bis 5 Gew.-% wenigstens eines monoethylenisch ungesättigten Monomers C, mit ionischen und/oder ionisierbaren funktionellen Gruppen,
iv) 0 bis 10 Gew.-% vernetzender Monomere,
v) 0 bis 20 Gew.-% weiterer, wasserlöslicher, von i) verschiedner Monomere
durch radikalische, wässrige Emulsionspolymerisation.

Die vorliegende Erfindung betrifft weiterhin die durch das erfindungsgemäße Verfahren erhältlichen Polymerisatdispersionen sowie deren Verwendung.

## Beschreibung

Die vorliegende Erfindung betrifft ein Verfahren zur Herstellung wässriger Copolymerisatdispersionen durch radikalische, wässrige Emulsionspolymerisation von Monomermischungen, die im wesentlichen nichtionische wasserlösliche Monomere mit N-Vinylgruppen (Monomere A) und hydrophobe Monomere enthalten.

Copolymerisate aus wasserlöslichen Monomeren, die N-Vinylgruppen enthalten und hydrophoben Monomeren sind im Prinzip bekannt. In der Regel erfolgt ihre Herstellung durch radikalische Lösungspolymerisation in einem organischen Lösungsmittel, beispielsweise einem aliphatischen Alkohol mit 1 bis 4 C-Atomen oder in Alkohol/Wasser-Mischungen (siehe beispielsweise US-A 4,520,179, US-A 5,319,041 oder EP-A 418721). Ein Verzicht auf organische Lösungsmittel ist jedoch nicht zuletzt aus Kostengründen und Gründen der besseren Umweltverträglichkeit grundsätzlich von Interesse.

Der Ersatz der organischen Lösungsmittel durch Wasser als Lösungsmittel ist bei der radikalischen Copolymerisation von wasserlöslichen Monomeren mit N-Vinylgruppen und hydrophoben Comonomeren nur in eingeschränktem Maße möglich. So beschreibt die DE-A 2218935 die radikalische Copolymerisation von N-Vinylpyrrolidon mit bis zu 30 Gew.-%, vorzugsweise bis zu 15 Gew.-% hydrophoben Monomeren im Sinne einer wässrigen Lösungspolymerisation. Bei der in der WO 93/18073 beschriebenen wässrigen Lösungspolymerisation von N-Vinyllactamen ist der Anteil an hydrophoben Monomeren ebenfalls auf unterhalb 30 Gew.-%, bezogen auf die Gesamtmenge der zu polymerisierenden Monomere, beschränkt.

Grundsätzlich sollten Polymere mit einem höheren Gehalt an hydrophoben Monomeren auf dem Wege der radikalischen, wässrigen Emulsionspolymerisation zugänglich sein. In der DE-A 4139963 sowie in der WO 93/15120 wird allerdings darauf hingewiesen, dass die radikalische, wässrige Emulsionspolymerisation von Monomermischungen, die mehr als 10 Gew.-% Vinylpyrrolidon enthalten, zu instabilen, sich trennenden, hochviskosen Dispersionen führt, die sich zudem schlecht reproduzieren lassen. Aus diesem Grund weicht man bei der Herstellung wässriger Polymerisatdispersionen, die größere Mengen wasserlösliche, N-Vinylgruppen enthaltende Monomere einpolymerisiert enthalten, auf eine radikalische Lösungspolymerisation in einem alkoholischen Lösungsmittel und anschließendem Austausch des Lösungsmittels gegen Wasser aus (siehe z. B. DE-A 4139963). Neben den oben genannten Nachteilen der Verwendung von Alkoholen als Lösungsmittel ist zu beachten, dass Alkohole im Unterschied zu Wasser als Regler in die radikalische Polymerisationsreaktion eingreifen, so dass Copolymere mit großen Molekulargewichten auf diesem Wege nicht zugänglich sind.

Die WO 93/15120 beschreibt Emulsionspfropfcopolymerisate, die durch radikalische Pfropfung hydrophober Monomere auf wasserlösliche Homo- oder Copolymere aus wasserlöslichen, N-Vinylgruppen enthaltenden Monomeren in wässriger Emulsion zugänglich sind.

Die US-A 4,167,439 beschreibt nichtionische Copolymere, die 5 bis 30 Gew.-% N-Vinylpyrrolidon, 15 bis 60 Gew.-% Acrylamid und 30 bis 70 Gew.-% Methylmethacrylat einpolymerisiert enthalten, und die durch radikalische Polymerisation in einem wässrigen Lösungsmittel erhältlich sind. Hierbei handelt es sich um sogenannte Mikroemulsionspolymerisate, deren Teilchengrößen vorzugsweise im Bereich von 0,05 bis 0,08 µm liegen. Die Verwendung größerer Mengen Acrylamid bei Polymerisationsreaktionen im wässrigen Medium ist jedoch nicht unproblematisch, da der Gehalt an nicht polymerisiertem Acrylamid in den hierbei erhältlichen Dispersionen vergleichsweise hoch ist und diese Verbindung im Verdacht steht, krebserregend oder zumindest allergen zu sein.

Die DE-A 43 42 281 beschreibt die Polymerisation von Monomermischungen, die im wesentlichen aus N-Vinylcaprolactam bestehen, in wässriger Emulsion. Da die Löslichkeit von Polymerisaten auf der Basis von N-Vinylcaprolactam, insbesondere bei den bevorzugten Gehalten von einpolymerisiertem N-Vinylcaprolactam oberhalb 90 Gew.-%, mit steigender Temperatur in Wasser abnimmt, findet die Polymerisation zwar in wässriger Emulsion statt, die erhältlichen Polymerisate sind jedoch klar wasserlöslich. Derartige Polymerisate sind nicht Gegenstand der vorliegenden Erfindung.

Der vorliegenden Erfindung liegt somit die Aufgabe zugrunde, ein Verfahren zur Herstellung von Copolymerisaten aus wasserlöslichen Monomeren A, die eine N-Vinylgruppe aufweisen, und wenigstens 30 Gew.-% hydrophoben Monomeren B, durch radikalische Polymerisation der Monomere B in einem Reaktionsmedium, das im wesentlichen aus Wasser besteht, bereitzustellen. Es wurde nun überraschenderweise gefunden, dass diese Aufgabe gelöst werden kann, wenn man die Polymerisation als radikalische, wässrige Emulsionspolymerisation unter Verwendung eines in Wasser löslichen Polymerisationsinitiators durchführt.

Dementsprechend betrifft die vorliegende Erfindung ein Verfahren zur Herstellung wässriger Copolymerisatdispersion durch radikalische, wässrige Emulsionspolymerisation ethylenisch ungesättigter Monomere, umfassend
i) 10 bis 70 Gew.-% wenigstens eines nichtionischen Monomers A mit einer Wasserlöslichkeit oberhalb 60 g/l bei 25 °C, das eine N-Vinylgruppe aufweist,
ii) 30 bis 90 Gew.-% wenigstens eines monoethylenisch ungesättigten, hydrophoben Monomers B mit einer Wasserlöslichkeit unterhalb 60 g/l bei 25 °C sowie gegebenenfalls
iii) 0 bis 5 Gew.-% wenigstens eines monoethylenisch ungesättigten Monomers C, das wenigstens eine ionische und/oder ionisierbare funktionelle Gruppe aufweist,
iv) 0 bis 10 Gew.-% wenigstens eines Monomers D, das wenigstens 2 ethylenisch ungesättigte Bindungen aufweist,
v) 0 bis 20 Gew.-% wenigstens eines weiteren, von den Monomeren A und C verschiedenen monoethylenisch ungesättigten Monomers E mit einer Wasserlöslichkeit >60 g/l bei 25 °C,
das dadurch gekennzeichnet ist, dass man einen in Wasser löslichen Polymerisationsinitiator verwendet. Die für die Monomere A bis E hier und im folgenden angegebenen Mengen in Gew.-% beziehen sich stets auf die Gesamtmenge der zu polymerisierenden Monomere.

Die vorliegende Erfindung betrifft weiterhin die durch das erfindungsgemäße Verfahren erhältlichen Polymerisatdispersionen.

Grundsätzlich werden solche wasserlöslichen Initiatoren verwendet, deren Wasserlöslichkeit so groß ist, dass die verwendete Initiatormenge im jeweiligen Reaktionsmedium vollständig gelöst vorliegt. Bevorzugte Polymerisationsinitiatoren umfassen wasserlösliche Azoverbindungen wie 2,2'-Azobis-[2-(2-imidazolin-2-yl)propan, 2,2'-Azobis-(2-amidinopropan) und deren Säureadditionssalze, insbesondere deren Hydrochloride, Acetate oder (Hydrogen)sulfate, 4,4'-Azobis(4-cyanovaleriansäure) und deren Alkalimetall- oder Ammoniumsalze, insbesondere deren Natriumsalze, oder 2-(Carbamoylazo)isobutyronitril. Sie umfassen weiterhin wasserlösliche Peroxide und Hydrogenperoxide wie tert.-Butylhydroperoxid, tert.-Amylhydroperoxid, Cumolhydroperoxid, Pinanhydroperoxid, Peroxodischwefelsäure und ihre Salze, insbesondere ihre Alkalimetall- oder Ammoniumsalze sowie Wasserstoffperoxid.

Die genannten Peroxide und Hydrogenperoxide können alleine oder vorzugsweise mit einem Reduktionsmittel, z. B. einem Salz der Hydroxymethansulfinsäure oder Ascorbinsäure oder einer Übergangsmetallverbindung, deren Übergangsmetall in wässriger Lösung in verschiedenen Oxidationsstufen vorliegen kann, beispielsweise Eisen(II)-Salzen oder Kupfer(II)-Salzen (sogenannte Redoxinitiatorsysteme) eingesetzt werden. Besonders bevorzugte Initiatoren sind die oben genannten wasserlöslichen Azoverbindungen, insbesondere solche, die in Salzform vorliegen oder in der Lage sind Salze zu bilden, die Salze der Peroxodischwefelsäure und Wasserstoffperoxid, wobei die letztgenannten Initiatoren vorzugsweise zusammen mit Eisen(II)- Salzen oder Kupfer(II)-Salzen verwendet werden.

Die für die Polymerisation verwendete Initiatormenge liegt vorzugsweise im Bereich von 0,02 bis 15 Mol-%, insbesondere 0,05 bis 10 Mol-% und ganz besonders bevorzugt 0,1 bis 3 Mol-%, bezogen auf die Gesamtmenge der zu polymerisierenden Monomere A bis E. Bei Verwendung der oben genannten Azoverbindungen als Initiatoren liegt die Initiatormenge vorzugsweise unterhalb 1 Mol-%, wohingegen bei den Peroxiden und Hydroperoxiden als Polymerisationsinitiatoren vorzugsweise größere Mengen verwendet werden. Vorzugsweise wird der Polymerisationsinitiator in gelöster bzw. verdünnter Form der Polymerisationsreaktion zugeführt. Als Lösungsmittel kommen die. o. g. C₁-C₄-Alkohole und/oder Wasser in Frage, wobei vorzugsweise Wasser als alleiniges Lösungs- bzw. Verdünnungsmittel verwendet wird. Der Initiatorgehalt solcher Lösungen liegt vorzugsweise im Bereich von 0,2 bis 20 Gew.-% und insbesondere im Bereich von 0,5 bis 10 Gew.-%.

Der Initiator kann im Polymerisationsansatz vorgelegt oder nach Maßgabe seines Verbrauchs zugeführt werden. In einer bevorzugten Ausführungsform der vorliegenden Erfindung werden wenigstens 70 %, insbesondere wenigstens 80 % und ganz besonders bevorzugt wenigstens 90 % des Initiators als wässrige oder wässrig-alkoholische Lösung kontinuierlich der Polymerisationsreaktion zugeführt. Eine kleine Initiatormenge, vorzugsweise wenigstens 1 %, insbesondere wenigstens 2 % und ganz besonders bevorzugt wenigstens 5 % des Initiators werden im Reaktor vorgelegt, um die Polymerisationsreaktion zu starten.

Als Reaktionsmedium für die radikalische Emulsionspolymerisation dient Wasser, das in der Regel nicht mehr als 20 Gew.-%, vorzugsweise nicht mehr als 10 Gew.-%, und insbesondere nicht mehr als 5 Gew.-%, bezogen auf das Reaktionsmedium, einen oder mehrere C₁-C₄-Alkohole, wie Methanol, Ethanol, n-Propanol, n-Butanol oder i-Butanol enthält. Besonders bevorzugt wird Wasser als alleiniges Reaktionsmedium verwendet. Die Polymerisation wird üblicherweise bei einem nahezu neutralen pH-Wert, vorzugsweise im Bereich von pH 5 bis pH 9 durchgeführt. Dabei kann der pH-Wert durch Zugabe einer Base, wie Ammoniak, Natriumhydroxid oder einer Säure, wie Salzsäure oder Schwefelsäure eingestellt bzw. aufrechterhalten werden. Alternativ kann die Polymerisation in Gegenwart eines geeigneten Puffers, beispielsweise Ammoniumhydrogencarbonat, Hydrogenphosphat, Borat, Acetat, Citrat, Succinat, Glycinat oder Phthalat durchgeführt werden. Bevorzugt werden organische Puffersubstanzen, insbesondere allerdings Ammoniumhydrogencarbonat.

Die zu polymerisierenden Monomere können im wässrigen Reaktionsmedium vorgelegt werden (Batch-Verfahren). Vorzugsweise erfolgt die Polymerisation nach einem Zulaufverfahren. Hierunter ist zu verstehen, dass man die Hauptmenge, insbesondere Wenigstens 70 % und ganz besonders bevorzugt 75 bis 90 % der zu polymerisierenden Monomere, gegebenenfalls als wässrige oder wässrig-alkoholische Lösung oder als wässrige Emulsion dem Polymerisationsansatz zudosiert.

Bei einem Zulaufverfahren kann der Polymerisationsinitiator sowohl im Reaktionsgefäß vorgelegt oder in der oben beschriebenen Weise dem Polymerisationsansatz zudosiert werden. Vorzugsweise erfolgt die Zugabe des Initiators kontinuierlich und parallel zur Zugabe der Monomere. Ganz besonders bevorzugt werden die oben angegebenen Teilmengen der Monomere und des Initiators im wässrigen oder wässrig-alkoholischen Reaktionsmedium vorgelegt und auf Reaktionstemperatur gebracht. Die Zugabe der Monomere erfolgt in der Regel über einen Zeitraum von 0,5 bis 14 Stunden, vorzugsweise 1 bis 12 Stunden und ganz besonders bevorzugt innerhalb 2 bis 10 Stunden. Die Zugabe des Initiators erstreckt sich über einen gleichen oder vorzugsweise längeren Zeitraum.

Die Reaktionstemperatur liegt üblicherweise im Bereich von 60 bis 90 °C, kann jedoch auch bis 130 °C betragen. Die Umsetzung kann bei Normaldruck oder bei Anwendung höherer Temperaturen unter Eigendampfdruck oder unter Schutzgasüberdruck durchgeführt werden. Als Schutzgas kommt insbesondere Stickstoff in Betracht.

Die Polymerisation kann auch durch energiereiche Strahlung, z. B. γ-Strahlung initiiert werden oder im Sinne einer Photopolymerisation, d. h. durch Initiierung mit sichtbarem Licht oder UV-Licht in Gegenwart geeigneter, wasserlöslicher Photoinitiatoren durchgeführt werden. Geeignete Photoinitiatoren umfassen insbesondere wasserlösliche Derivate des Acetophenons, Benzophenons oder Thioxanthons, die in der Regel funktionelle Gruppen, die deren Wasserlöslichkeit6 verbessern, wie OH, Carboxyl, Nitro oder Aminogruppen tragen.

Vorzugsweise erfolgt im Anschluss an die eigentliche Polymerisation eine Nachpolymerisation zur Vervollständigung des Monomerumsatzes. Hierunter ist zu verstehen, dass man im Anschluss an die Zugabe der Monomere/des Polymerisationsinitiators weitere, Radikale bildende Initiatoren dem Polymerisationsansatz zuführt und gegebenenfalls die Temperatur der Reaktionsmischung auf Temperaturen oberhalb der eigentlichen Polymerisationstemperatur erhöht. Geeignete Initiatoren für die Nachpolymerisation sind die oben angegebenen Initiatoren sowie Percarbonate oder Peroxoester. Die genannten Peroxide bzw. Hydroperoxide werden vorzugsweise zusammen mit einem Reduktionsmittel und/oder einem Übergangsmetall (siehe oben) eingesetzt. Die Nachpolymerisation schließt sich in der Regel direkt oder innerhalb eines Zeitraums von bis zu 4 Stunden nach Beendigung der Monomer/Initiator-Zugabe an. Der für die Nachpolymerisation erforderliche Initiator kann auf einmal oder über einen Zeitraum von bis zu 10 Stunden zugegeben werden. Auch ist es möglich, für die Nachpolymerisation mehrere Initiatoren nacheinander zuzugeben.

Die erhaltenen Dispersionen können im Anschluss an die Polymerisation, gegebenenfalls anstelle einer Nachpolymerisation, einer physikalischen Nachbehandlung, beispielsweise einer Wasserdampfdestillation oder dem Strippen mit einem inerten Gas, beispielsweise Stickstoff, unterworfen werden. Hierdurch werden mit Wasserdampf flüchtige Verunreinigungen, beispielsweise Restmonomere aus der Dispersion entfernt.

Die durch das erfindungsgemäße Polymerisationsverfahren erhaltenen Polymerisate weisen in der Regel relativ hohe Molekulargewichte auf. Sind niedrigere Molekulargewichte erwünscht, so können diese durch Zusatz eines Reglers zum Polymerisationsansatz eingestellt werden.

Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten. Hierbei handelt es sich beispielsweise um Di-n-butylsulfid, Di-n-octylsulfid, Diphenylsulfid, Diisopropyldisulfid, Di-n-butyldisulfid, Di-n-hexyldisulfid, Diacetyldisulfid und Di-tert.-butyltrisulfid. Vorzugsweise enthalten die Regler Schwefel in Form von SH-Gruppen. Beispiele für solche Regler sind n-Butylmercaptan, n-Hexylmercaptan oder n-Dodecylmercaptan. Besonders bevorzugt werden wasserlösliche, schwefelhaltige Polymerisationsregler, wie beispielsweise Hydrogensulfite, Disulfite und Verbindungen wie Ethylthioglykolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Diethanolsulfid, Thiodiglykol, Ethylthioethanol, Thioharnstoff und Dimethylsulfoxid. Weiterhin eignen sich als Regler Allylverbindungen, wie Allylalkohol oder Allylbromid, Benzylverbindungen, wie Benzylchlorid oder Alkylhalogenide, wie Chloroform, Bromtrichlormethan oder Tetrachlormethan. In einer bevorzugten Ausführungsform wird der Regler, gegebenenfalls als Lösung in Wasser und/oder einem C₁-C₄-Alkohol dem Reaktionsansatz zudosiert.

Das erfindungsgemäße Verfahren wird gegebenenfalls unter Verwendung der bei einer Emulsionspolymerisation üblichen oberflächenaktiven Substanzen, d. h. Emulgatoren und/oder Schutzkolloide, durchgeführt. Bei Verwendung oberflächenaktiver Substanzen werden diese in der Regel in Mengen bis zu 20 Gew.-%, vorzugsweise 0,5 bis 10 Gew.-% und insbesondere 1 bis 5 Gew.-%, bezogen auf die zu polymerisierenden Monomere, verwendet. Geeignete oberflächenaktive Verbindungen umfassen sowohl Schutzkolloide als auch neutrale, anionische oder kationische Emulgatoren. Geeignete anionische Emulgatoren umfassen insbesondere die Alkalimetall- oder Ammoniumsalze von längerkettigen Fettsäuren, von Schwefelsäurehalbestern ethoxylierter Fettalkohole (EO-Grad: 4 bis 30, Alkylrest: C₁₀-C₂₂) und ethoxylierter Alkylphenole (EO-Grad: 3 bis 50, Alkylrest: C₄-C₁₀), von Alkylsulfonsäuren (Alkylrest: C₁₂-C₁₈) und von Alkylarylsulfonsäuren (Alkylrest: C₉-C₁₈). Ferner können als anionische Emulgatoren die Alkalimetallsalze von Sulfobernsteinsäuredialkylestern sowie die Alkalimetallsalze der Sulfonsäuren von Alkylnaphthalinen und von Naphthalin verwendet werden. Ferner können weiterhin kationenaktive Verbindungen, wie quartäre Fettamine, quartäre Alkylpyridine (Alkylrest C₈-C₃₀), quartäre N-Alkylmorpholine (Alkylrest C₈-C₃₀) oder alkylierte Imidazoline verwendet werden.

Zusammen mit den Emulgatoren können zusätzliche, emulgierende wirkende Hilfsmittel (Cosolvenzien) in Mengen von bis zu 20 Gew.-%, vorzugsweise 0,5 bis 10 Gew.-% und insbesondere 1 bis 5 Gew.-%, bezogen auf die Monomeren, zugesetzt werden. Geeignete Cosolvenzien umfassen lineare oder verzweigte aliphatische oder cycloaliphatische C₁-C₃₀-Alkohole oder Mischungen davon, z. B. n-Butanol, n-Hexanol, Cyclohexanol, 2-Ethylhexanol, Isooctanol, n-Octanol, n-Decanol, n-Dodecanol, Stearylalkohol, Oleylalkohol oder Cholesterin. Weiterhin sind als Cosolvenzien C₄-C₂₀-Alkandiole, Ethylenglykolalkylether mit 1 bis 4 Ethylenoxideinheiten, z. B. Ethylenglykolmonobutylether, Diethylenglykolmonoethylether oder Tetraethylenglykoldimethylether sowie N-Alkylpyrrolidone, N-Alkyl- und N,N-Dialkylacetamide mit jeweils 1 bis 8 Kohlenstoffatomen in der Alkylkette, z. B. N-Methylpyrrolidon, N-Hexylpyrrolidon, Diethylacetamid oder N-Octylacetamid.

Das erfindungsgemäße Verfahren eignet sich insbesondere für die Herstellung wässriger Polymerisatdispersionen, die 20 bis 60 Gew.-% und insbesondere 30 bis 60 Gew.-% Monomere A, bezogen auf die Gesamtmenge der zu polymerisierenden Monomere, einpolymerisiert enthalten. Bei den Monomeren A handelt es sich vorzugsweise um N-Vinyllactame mit 6 bis 8 C-Atomen, z. B. N-Vinylpyrrolidon, N-Vinylpiperidon, N-Vinylcaprolactam und/oder um acyclische N-Vinylcarbonsäureamide mit 2 bis 6 C-Atomen, z. B. N-Vinylformamid oder N-Methyl-N-vinylacetamid. Besonders bevorzugte Monomere A sind die N-Vinyllactame. N-Vinylimidazol ist als Monomer A ebenfalls geeignet. In einer bevorzugten Ausführungsform der vorliegenden Erfindung ist die Wasserlöslichkeit des Monomers A > 100 g/l (bei 25 °C).

Als Monomere B kommen grundsätzlich alle hydrophoben Monomere mit einer Wasserlöslichkeit unterhalb 60 g/l bei 25 °C in Frage, die mit den Monomeren A copolymerisierbar sind. Hierbei handelt es sich insbesondere um die C₁-C₁₀-Alkylester monoethylenisch ungesättigter C₃-C₆-Carbonsäuren, insbesondere die Ester der Acrylsäure und der Methacrylsäure mit C₁-C₁₀-Alkanolen oder C₅-C₁₀-Cycloalkanolen wie Methanol, Ethanol, n-Propanol, Isopropanol, n-Butanol, Isobutanol, 2-Butanol, tert.-Butanol, n-Pentanol, n-Hexanol, 2-Ethylhexan-1-ol, n-Octanol, n-Decanol, 2-Propylheptan-1-ol, Cyclohexanol, 4-tert.-Butylhexanol oder 2,3,5-Trimethylcyclohexanol. Weiterhin kommen als Monomere B die Di-C₁-C₁₀-alkylester ethylenisch ungesättigter Dicarbonsäuren wie Maleinsäure, Fumarsäure oder Itaconsäure mit den oben genannten C₁-C₁₀-Alkanolen oder C₅-C₁₀-Cycloalkanolen, z. B. Maleinsäuredimethylester oder Maleinsäuredi-n-butylester in Frage. Weiterhin kommen als Monomere B vinylaromatische Verbindungen wie Styrol und α-Methylstyrol in Frage, die gegebenenfalls am aromatischen Ring einen oder mehrere Substituenten aufweisen können, die ausgewählt sind unter C₁-C₄-Alkyl, Halogenatomen, insbesondere Chlor, und/oder Hydroxylgruppen, die gegebenenfalls auch ethoxyliert sein können. Weiterhin umfassen die Monomere B die Vinyl-, Allyl- und Methallylester linearer oder verzweigter, aliphatischer Carbonsäuren mit 2 bis 20 C-Atomen wie Vinylacetat, Vinylpropionat, Vinylbutyrat, Vinylvalerat, Vinylhexanoat, Vinyl-2-ethylhexanoat, Vinyldecanoat, Vinyllaurat und Vinylstearat sowie die entsprechenden Allyl- und Methallylester. Geeignete Monomere B sind weiterhin die Vinyl-, Allyl- und Methallylether linearer oder verzweigter, aliphatischer Alkohole mit 2 bis 20 C-Atomen, z. B. Vinylmethylether, Vinylethylether, Vinyldodecylether, Vinylhexadecylether und Vinylstearylether. Vorzugsweise werden die Monomere B in Mengen von 40 bis 70 Gew.-% und insbesondere in Mengen von 40 bis 60 Gew.% verwendet.

Als Monomere C, die wenigsten eine ionische und/oder ionisierbare funktionelle Gruppen aufweisen, kommen sowohl anionische bzw. saure Monomere als auch kationische Monomere in Frage. Die Monomere C werden vorzugsweise in Mengen bis 5 Gew.-% und insbesondere bis 3 Gew.-% - bei den sauren Monomeren auf die freie Säure bezogen - verwendet. Geeignete anionische bzw. saure Monomere umfassen insbesondere solche Verbindungen, die wenigstens eine Carboxylgruppe, Sulfonsäuregruppe und/oder Phosphonsäuregruppe im Molekül aufweisen. Geeignete anionische bzw. saure Monomere umfassen ethylenisch ungesättigte C₃-C₆-Monocarbonsäuren wie Acrylsäure, Methacrylsäure oder Crotonsäure, ethylenisch ungesättigte C₄-C₈-Dicarbonsäuren wie Maleinsäure, Fumarsäure, Itaconsäure oder Methylenmalonsäure sowie die Halbester der vorgenannten C₄-C₈-Dicarbonsäuren mit C₁-C₁₀-Alkanolen wie Monomethylmaleinsäureester, Mono-n-butylmaleinsäureester; ethylenisch ungesättigte Sulfonsäuren wie Vinylsulfonsäure, Styrolsulfonsäure, 2-Acrylamido-3-methylpropansulfonsäure und 2-Methacrylamido-2-methylpropansulfonsäure und ethylenisch ungesättigte Phosphonsäuren wie Vinylphosphonsäure. Die genannten Säuren werden vorzugsweise in ihrer Salzform eingesetzt. Geeignete Gegenionen umfassen Alkalimetall- und Erdalkalimetallionen wie Natrium, Kalium oder Calcium sowie Ammoniumionen. In einer bevorzugten Ausführungsform der vorliegenden Erfindung werden die freien Säuren vor der Polymerisation mit Hilfe einer geeigneten Base, vorzugsweise in Form einer wässrigen oder wässrigalkoholischen Lösung, in die anionische Form überführt. Geeignete Basen umfassen die Hydroxide und Carbonate der vorgenannten Alkalimetalle, Calciumhydroxid, Ammoniak und organische Amine, Pyridine und Amidine. Geeignete organische Amine umfassen insbesondere Mono-, Di- oder Trialkanolamine mit 2 bis 5 C-Atomen im Alkanolrest wie Mono-, Di- oder Triethanolamin, Mono-, Di- oder Tri(iso)propanolamin oder 2-Amino-2-methyl-propanol; Alkandiolamine mit 2 bis 4 C-Atomen im Alkandiolrest wie 2-Amino-2-methyl-1,3-propandiol oder 2-Amino-2-ethyl-1,3-propandiol; Alkanpolyolamine, wie Di(2-ethylhexyl)amin, Triamylamin oder Dodecylamin und Aminoether wie Morpholin.

Geeignete kationische Monomere C umfassen ethylenisch ungesättigte, stickstoffbasische Verbindungen, z. B. N-Vinylimidazole wie N-Vinylimidazol, 2-Methyl-1-vinylimidazol, 2-Ethyl-1-vinylimidazol, 4-Methyl-1-vinylimidazol oder 5-Methyl-N-vinylimidazol, N-Vinylimidazoline, 2-, 3- oder 4-Vinylpyridin, die durch Alkylierung in die quartäre Form überführt wurden. Geeignete Akylierungsmittel umfassen Alkylhalogenide wie Methylchlorid, Methylbromid, Methyliodid, Ethylchlorid, Propylchlorid, Benzylchlorid oder Benzylbromid; weiterhin Dialkylsulfate, insbesondere Dimethylsulfat oder Diethylsulfat sowie Alkylenoxide wie Ethylenoxid oder Propylenoxid in Gegenwart von Säuren. Bevorzugte Alkylierungsmittel sind Methylchlorid, Dimethylsulfat oder Diethylsulfat. Weiterhin umfassen die kationischen Monomere C auch Diallylammoniumverbindungen, wie Dimethyldiallylammoniumchlorid, Diethyldiallylammoniumchlorid oder Diallylpiperidiniumbromid. Darüber hinaus können als kationische Monomere C die Ester ethylenisch ungesättigter C₃-C₆-Carbonsäuren mit Aminoalkanolen der allgemeinen Formel I oder die Amide der ethylenisch ungesättigten Carbonsäuren mit Aminen der allgemeinen Formel II 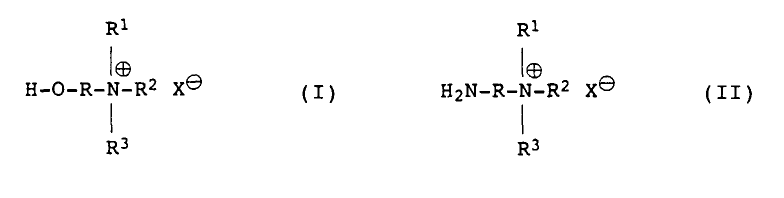 verwendet werden. Hierin stehen R für C₂-C₅-Alkylen, R¹, R², R³ unabhängig voneinander für CH₃, C₂H₅, C₃H₇ und X^{⊖} für ein Anion einer Mineralsäure, beispielsweise für Chlorid, für das Anion einer Carbonsäure oder für Methosulfat oder Ethosulfat.

Weiterhin können die erfindungsgemäßen Polymerisatdispersionen unter Verwendung sogenannter vernetzender Monomere D, d. h. Monomere, die wenigstens zwei ethylenisch ungesättigte Bindungen aufweisen, hergestellt werden. Geeignete Monomere D umfassen insbesondere die Di- oder Polyester zweiwertiger oder höher wertiger Alkohole mit ethylenisch ungesättigten C₃-C₆-Carbonsäuren. Beispiele für derartige Verbindungen sind Alkylenglykoldiacrylate und Dimethacrylate wie Ethylenglykoldi(meth)acrylat, 1,3- oder 1,4-Butylenglykoldi(meth)acrylat, Diethylenglykoldi(meth)acrylat, 1,6-Hexandioldi(meth)acrylat, Neopentylglykoldi(meth)acrylat, Triethylenglykoldi(meth)acrylat, Tetraethylenglykoldi(meth)acrylat, 1,12-Dodecandioldi(meth)acrylat, Polyethylenglykoldi(meth)acrylat, 2,2-Bis(p-(meth)acryloxyphenyl)propan, Tripropylenglykoldi(meth)acrylat, Trimethylolpropantri(meth)acrylat, Pentaerythrittri(meth)acrylat oder Pentaerythrittetra(meth)acrylat. Weiterhin kommen als Monomere D die Vinyl-, Allyl- und Methallylester ethylenisch ungesättigter C₃-C₆-Carbonsäuren wie Vinyl(meth)acrylat, Allyl(meth)acrylat und Methallyl(meth)acrylat, die Vinyl-, Allyl- und Methallylester aliphatischer oder aromatischer Dicarbonsäuren wie Divinylphthalat oder Diallylphthalat, polyfunktionelle Amide ethylenisch ungesättigter Carbonsäuren, insbesondere N,N'-Methylenbisacrylamid, N,N'-Butylidenbisacrylamid, Bis(acrylamido)essigsäuremethylester, Terephthalylidentetraacrylamid, in Frage. Ferner kommen als Monomere D Divinylaromaten, wie Divinylbenzol sowie Divinyl-, Diallyl- oder Dimethallylderivate des Harnstoffs bzw. des Imidazolidons, wie N,N'-Divinylharnstoff und N,N'-Divinylimidazolidin-1-on in Betracht. Die Monomere D werden vorzugsweise in Mengen bis 5 Gew.-% und vorzugsweise bis 2 Gew.-% verwendet.

Als Monomere E kommen neutrale wasserlösliche Monomere in Frage, die keine N-Vinylgruppe aufweisen. Hierzu zählen Acrylnitril, die Hydroxyalkylester der oben genannten C₃-C₆-Carbonsäuren, wie Hydroxyethyl(meth)acrylat, 2- oder 3-Hydroxypropyl(meth)acrylat, 4-Hydroxybutyl(meth)acrylat, die Amide und N-Methylolamide ethylenisch ungesättigter C₃-C₆-Carbonsäuren wie Acrylamid und Methacrylamid sowie die Anhydride ethylenisch ungesättigter C₄-C₆-Dicarbonsäuren wie Maleinsäureanhydrid. Die Monomere E werden vorzugsweise in Mengen bis 10 Gew.% verwendet.

In einer bevorzugten Ausführungsform der vorliegenden Erfindung wird die Polymerisation in Abwesenheit der Monomere C durchgeführt. Dementsprechend weist die Monomermischung der zu polymerisierenden Monomere folgende Zusammensetzung auf:
- 10 bis 70 Gew.-%, vorzugsweise 20 bis 60 Gew.-% und insbesondere 30 bis 60 Gew.-% Monomere A
- 30 bis 90 Gew.-%, vorzugsweise 40 bis 80 Gew.-% und insbesondere 40 bis 70 Gew.-% Monomere B
- 0 bis 10 Gew.-%, vorzugsweise weniger als 5 Gew.-%, insbesondere weniger als 2 Gew.-% vernetzende Monomere D
- 0 bis 20 Gew.-%, insbesondere weniger als 10 Gew.-% und besonders bevorzugt keine weiteren, von den Monomeren A verschiedenen wasserlöslichen Monomeren E.

Ganz besonders bevorzugt umfaßt die zu polymerisierende Monomermischung in dieser Ausführungsform nur Monomere A und B.

In diesem Fall kann die Polymerisation sowohl in Gegenwart von oberflächenaktiven Verbindungen als auch frei von oberflächenaktiven Verbindungen durchgeführt werden. Die letztere Variante wird generell bevorzugt, insbesondere dann, wenn die Wasserlöslichkeit der hydrophoben Monomere B oberhalb 10 g/l liegt. Sofern für den Anwendungszweck erforderlich, können jedoch auch derartige Monomermischungen in Gegenwart der oben genannten Emulgatoren und/oder Schutzkolloide polymerisiert werden.

In einer anderen Ausführungsform der vorliegenden Erfindung werden die Monomere A und B sowie gegebenenfalls die Monomere D und E in Gegenwart von ionischen oder ionisierbaren Monomeren C polymerisiert. Vorzugsweise werden 0,1 bis 10 Gew.-%, insbesondere 0,2 bis 5 Gew.-% und besonders bevorzugt 0,5 bis 3 Gew.-% Monomere C, bezogen auf die Gesamtmenge der zu polymerisierenden Monomere verwendet. Die Monomere C bewirken eine Stabilisierung der Polymerisate, so dass die Verwendung von Emulgatoren und/oder Schutzkolloiden bei der Emulsionspolymerisation überflüssig wird. Sofern für den Anwendungszweck gewünscht, können jedoch auch derartige oberflächenaktive Verbindungen der Polymerisationsreaktion in den oben angegebenen Mengen zugesetzt werden. Bevorzugt ist jedoch das von oberflächenaktiven Verbindungen freie Herstellungsverfahren. Hinsichtlich der Mengen der Monomere A, B, D und E gilt das oben gesagte. Bevorzugte Monomermischungen dieser Ausführungsform umfassen nur die Monomere A bis C.

Die durch das erfindungsgemäße Verfahren erhältlichen Polymerisatdispersionen sind neu und ebenfalls Gegenstand der vorliegenden Erfindung. Der Feststoffgehalt derartiger Polymerisatdispersionen liegt üblicherweise im Bereich von 10 bis 60 Gew.-%, vorzugsweise 15 bis 40 Gew.-%, bezogen auf das Gesamtgewicht der Dispersion. Die Lichtdurchlässigkeit der erfindungsgemäß erhältlichen Polymerisatdispersionen (bezogen auf 0,5 Gew.-% Verdünnung bei 1 cm Schichtdicke) liegt in der Regel oberhalb 50 %, vorzugsweise oberhalb 70 %. Die Teilchengröße der dispergierten Polymerisatteilchen liegt in der Regel oberhalb 50 nm und vorzugsweise oberhalb 100 nm. Als Bezugsgröße wird hierbei das Gewichtsmittel der Teilchengröße gewählt, wie es mittels einer analytischen Ultrazentrifuge entsprechend den Methoden von W. Scholtan und H. Langen, Kolloid-Z.u.Z. Polymere 250 (1972) 782 - 796 bestimmt werden kann.

In der Regel zeichnen sich die erfindungsgemäß erhältlichen Polymerisate durch hohe Molekulargewichte aus, entsprechend K-Werten nach Fikentscher (H. Fikentscher, Cellulose-Chemie, Band 13, 1932, S. 58 - 64; gemessen als 1 gew.-%ige Lösung des Polymerisats in Ethanol) oberhalb 50, vorzugsweise oberhalb 70 und besonders bevorzugt oberhalb 80. Durch Einsatz von Molekulargewichtsreglern können auch niedrigere Molekulargewichte eingestellt werden. In der Regel ist auch in diesen Fällen das gewichtsmittlere Molekulargewicht M_{w} > 50 000, entsprechend K-Werten nach Fikentscher oberhalb 30.

Die gemäß dem erfindungsgemäßen Verfahren erhältlichen Polymerisatdispersionen können, sofern erforderlich, durch ein dem Stand der Technik entsprechendes Trocknungsverfahren in feste Pulver überführt werden. Als Trocknungsverfahren kommen neben der Gefriertrocknung insbesondere die Sprühtrocknung, die Sprühwirbelschichttrocknung, die Walzentrocknung und die Bandtrocknung in Frage. Für eine Reihe von Anwendungen empfiehlt es sich, die wässrigen Polymerisatdispersionen mit Hilfe von Verdampferextrudern in feste Formen zu überführen.

Die nach dem erfindungsgemäßen Verfahren erhaltenen Polymerisate wirken einerseits in wässrigem Medium als Verdicker und sind andererseits in der Lage, wasserlösliche Filme zu bilden. Sie sind brauchbar als Hilfsstoffe für pharmazeutische, kosmetische oder agrochemische Zubereitungen und zur Herstellung von Anstrich- und Beschichtungsmitteln, Leimen und Klebstoffen. Insbesondere kommen sie zur Anwendung in kosmetischen und pharmazeutischen Zubereitungen, beispielsweise als Additive oder Träger in Haarlack, Haarfestiger oder Haarspray; in hautkosmetischen Zubereitungen, als Hautklebegele oder als Immunochemikalien, z. B. als Katheter-Coatings. Spezielle pharmazeutische Anwendungen der erfindungsgemäßen Polymerisate umfassen insbesondere den Einsatz als Feucht- oder Trockenbindemittel, Matrixretardierungsmittel oder Coatingretardierungsmittel (für Slow-Release-Darreichungsformen), Gelbildner, Instant-Release-Coatings und Dragierhilfsmittel. Weiterhin können die erfindungsgemäß hergestellten Polymerisate als Hilfsstoffe für die Agrochemie, beispielsweise für das Saatgut-Coating oder für Soil-Release-Düngemittelformulierungen oder als Hilfsmittel bei der Herstellung von Fischfuttergranulaten verwendet werden.

Aufgrund der hohen Dispergierwirkung der erfindungsgemäß hergestellten Polymerisate, sowohl für organische als auch für anorganische Pigmente, kommen die erfindungsgemäßen Polymerisate als Rostverhinderer oder Rostentferner von metallischen Oberflächen, als Kesselsteinverhinderer oder Kesselsteinentferner, als Dispergiermittel in Farbstoffpigmentdispersionen, beispielsweise in Druckfarben, in Frage. In diesem Zusammenhang sei auf die Verwendung der erfindungsgemäßen Polymerisate für Tintenstrahl-Aufzeichnungsmedien, Tinten- und Kugelschreiberpasten hingewiesen.

Anwendungstechnisch von Interesse ist weiterhin die hohe Neigung der erfindungsgemäßen Polymerisate, Komplexe mit organischen Verbindungen (z. B. niedere Kohlenwasserstoffe, Phenole, Tannin sowie diverse Antioxidantien), mit Enzymen und Proteinen, oder mit anderen organischen Polymeren zu bilden. Weiterhin bilden die erfindungsgemäßen Polymerisate Komplexe mit anorganischen Verbindungen, insbesondere mit Wasserstoffperoxid, Halogeniden, Metallen oder Metallsalzen. Dementsprechend können die erfindungsgemäßen Polymerisate zur Entfernung von Tannin, Phenolen, Eiweißstoffen oder polyvalenten Kationen aus wässrigem Medium, in Ionenaustauschern, zur Stabilisierung von Wasserstoffperoxid, beispielsweise in Desinfektionsmitteln, zur Stabilisierung von Antioxidantien, beispielsweise in Konservierungsmitteln, als polymerer Co-ligand für Metallkomplexe der reversiblen Sauerstoffabsorption oder für Katalysatoren verwendet werden. Die erfindungsgemäßen Polymerisate können weiterhin für die Stabilisierung von Metallkolloiden verwendet werden. In diesem Zusammenhang sei auch auf die Verwendung der erfindungsgemäßen Polymerisate als Edelmetall-Kristallisationskeime für die Silberfällung und als Stabilisator für Silberhalogenidemulsionen hingewiesen.

Die erfindungsgemäßen Polymerisate eignen sich weiterhin zur Modifizierung von Oberflächen- und Grenzflächeneigenschaften. Aufgrund der modifizierenden Wirkung für Oberflächen können die erfindungsgemäßen Mittel als Coatings, z. B. für Polyolefine, für Glas und Glasfasern verwendet werden. Aufgrund ihrer oberflächenaktiven Wirkung finden sie weiterhin als Schutzkolloide, beispielsweise bei der Stabilisierung von Metallkolloiden oder bei der radikalischen wässrigen Emulsionspolymerisation Verwendung. In diesem Zusammenhang sei auch auf die Verwendung der erfindungsgemäßen Polymerisate als Hilfsmittel bei der Erdölgewinnung aus ölhaltigem Wasser, als Hilfsmittel bei der Erdöl- und Erdgasförderung sowie dem Erdöl- und Erdgastransport hingewiesen. Weiterhin finden die erfindungsgemäßen Polymerisate Verwendung als Hilfsmittel bei der Reinigung von Abwässern, sei es als Flockungshilfsmittel oder bei der Entfernung von Farb- und Ölresten aus Abwasser. Weiterhin können die erfindungsgemäßen Polymerisate als Phasentransferkatalysatoren und als Löslichkeitsverbesserer verwendet werden.

Weiterhin finden die erfindungsgemäßen Polymerisate Verwendung bei der Anfärbung von Polyolefinen, als Farbmischungsinhibitoren für fotografische Diffusions-Transfer-Materialien, als Haftvermittler für Farbstoffe, als Hilfsmittel für die Lithografie, für das Foto-Imaging, für die Diazotypie, als Hilfsmittel für den Metallguß und die Metallhärtung, als Hilfsmittel für Metallquenchbäder, als Hilfsmittel bei der Gasanalytik, als Bestandteil in Keramikbindern, als Papierhilfsmittel für Spezialpapiere, als Bindemittel in Papierstreichfarben und als Bindemittelbestandteil in Gipsbinden.

Die erfindungsgemäßen Polymerisate eignen sich Weiterhin als Protonenleiter und können in elektrisch leitenden Schichten, z. B. bei Charge-Transfer-Kathoden, als Festelektrolyte, z. B. in Festbatterien wie Lithiumbatterien, eingesetzt werden. Aus den erfindungsgemäßen Polymerisaten können Kontaktlinsen, synthetische Fasern, Luftfilter, z. B. Zigarettenfilter, oder Membrane hergestellt werden. Weiterhin finden die erfindungsgemäßen Polymerisate Verwendung in wärmebeständigen Schichten, wärmeempfindlichen Schichten und wärmeempfindlichen Widerständen.

Die im folgenden aufgeführten Beispiele sollen die Erfindung verdeutlichen, ohne sie jedoch einzuschränken.

### Beispiele:

### I. Analytik

Zur Charakterisierung der Polymerisate wurde ihr K-Wert viskosimetrisch in der von H. Fikentscher beschriebenen Weise (siehe H. Fikentscher Cellulose Chemie 13 (1932) 58 - 64 und 71 - 74 sowie Encyclopedia of Chemical Technology, 2. Ausgabe, Kirk Othmer, Wiley & Sons, 1970, S. 427 - 428) bestimmt. Hierzu trocknete man eine Probe der jeweiligen Dispersion und bereitete aus dem Polymerisat eine 1 gew.-%ige Lösung in Ethanol.

Zur Bestimmung der Lichtdurchlässigkeit verdünnte man eine Probe der jeweiligen Dispersion mit entionisertem Wasser auf einen Feststoffgehalt von 0,5 Gew.-% und maß dann die optische Durchlässigkeit bei 1 cm Schichtdicke.

Der Restmonomergehalt wurde gaschromatographisch bestimmt.

### II. Herstellung der erfindungsgemäßen Copolymerisatdispersionen (Beispiele 1 bis 20)

### Beispiel 1

Dispersion aus 30 Gew.-% N-Vinylpyrrolidon und 70 Gew.-% Vinylacetat.

In einem Reaktor mit Rührer, Rückflußkühler, Gaseinlaß und zwei getrennten Zuläufen wurde eine Mischung aus
- 20 g: N-Vinylpyrrolidon,
- 50 g: Vinylacetat,
- 5 g: Starterzulauf 1,
- 1000 g: Wasser
vorgelegt. Die Vorlage wurde mit Stickstoff gespült und auf 70 °C Innentemperatur erwärmt. Anschließend wurden unter Aufrechterhaltung der Temperatur der Monomerzulauf und der Starterzulauf 1 gleichzeitig und mit konstanter Geschwindigkeit über einen Zeitraum von 8 Stunden zugegeben. Während der Polymerisation wurde die Reaktionslösung mit verdünnter Ammoniaklösung auf pH 6 bis 7 eingestellt. Anschließend erhöhte man die Innentemperatur auf 75 °C und gab den Starterzulauf 2 über einen Zeitraum von 6 Stunden bei Aufrechterhaltung der Temperatur zu. Die Temperatur wurde weitere 2 Stunden bei 75 °C gehalten. Anschließend wurde das Reaktionsgemisch einer Wasserdampfdestillation unterworfen. Etwa 100 g Destillat wurden aufgefangen und der Feststoffgehalt auf ca. 20 Gew.-% eingestellt. Erhalten wurde eine weiße, sedimentationsstabile Dispersion mit einem K-Wert von 95, einem Feststoffgehalt von 19,5 Gew.-% und einem Restmonomergehalt von 130 ppm N-Vinylpyrrolidon. Eine eingedampfte Probe war in Ethanol klar löslich.
- Monomerzulauf:: 70 g N-Vinylpyrrolidon, 160 g Vinylacetat
- Starterzulauf 1:: Lösung von 1 g 2,2'-Azobis(2-amidinopropan)dihydrochlorid in 100 g Wasser.
- Starterzulauf 2,:: Lösung von 1 g 2,2'-Azobis(2-amidinopropan)dihydrochlorid in 100 g Wasser.

Der pH-Wert der Vorlage, des Starterzulaufs 1 und Starterzulaufs 2 wurde mit verdünnter Ammoniaklösung auf pH 6 eingestellt.

### Beispiel 2

Dispersion aus 30 Gew.-% N-Vinylpyrrolidon und 70 Gew.-% Vinylacetat.

In einem Reaktor mit Rührer, Rückflußkühler, Gaseinlaß und zwei getrennten Zuläufen wurde eine Mischung aus
- 20 g: N-Vinylpyrrolidon,
- 50 g: Vinylacetat,
- 6 g: Ammoniumhydrogencarbonat,
- 5 g: Starterzulauf 1,
- 1000 g: Wasser
vorgelegt. Die Polymerisationsvorschrift entspricht der von Beispiel 1. Der pH-Wert wird während der Polymerisation durch Pufferzusatz (Ammoniumhydrogencarbonat) konstant gehalten (pH 5 - 7). Erhalten wurde eine weiße, sedimentationsstabile Dispersion mit einem K-Wert von 98, einem Feststoffgehalt von 19,7 Gew.-% und einem Restmonomergehalt von 70 ppm N-Vinylpyrrolidon. Eine eingedampfte Probe war in Ethanol klar löslich.
- Monomerzulauf:: 70 g N-Vinylpyrrolidon,
160 g Vinylacetat.

Starterzuläufe 1 und 2 entsprechen denen von Beispiel 1.

### Beispiel 3

Dispersion aus 30 Gew.-% N-Vinylpyrrolidon und 70 Gew.-% Vinylacetat (geregelte Fahrweise).

Man führte die Polymerisation wie in Beispiel 1 beschrieben aus. Abweichend von Beispiel 1 enthielt der Monomerzulauf zusätzlich 2 g Allylalkohol.

Erhalten wurde eine weiße, schwach gelblich verfärbte, sedimentationsstabile Dispersion mit einem K-Wert von 72, einem Feststoffgehalt von 19,1 Gew.-% und einem Restmonomergehalt von 550 ppm N-Vinylpyrrolidon. Eine eingedampfte Probe war in Ethanol klar löslich.

### Beispiel 4

Dispersion aus 30 Gew.-% N-Vinylpyrrolidon und 70 Gew.-% Vinylacetat (geregelte Fahrweise).

Man führte die Polymerisation wie in Beispiel 1 beschrieben aus. Abweichend von Beispiel 1 enthielt der Monomerzulauf zusätzlich 0,5 g Mercaptoethanol. Erhalten wurde eine weiße, schwach gelblich verfärbte, sedimentationsstabile Dispersion mit einem K-Wert von 72, einem Feststoffgehalt von 19,1 Gew.-% und einem Restmonomergehalt von 550 ppm N-Vinylpyrrolidon. Eine eingedampfte Probe war in Ethanol klar löslich.

### Beispiel 5

Dispersion aus 20 Gew.-% N-Vinylpyrrolidon und 80 Gew.-% Vinylacetat (geregelte Fahrweise).

In einem Reaktor mit Rührer, Rückflußkühler, Gaseinlaß und zwei getrennten Zuläufen wurde eine Mischung aus
- 15 g: N-Vinylpyrrolidon,
- 50 g: Vinylacetat,
- 5 g: Starterzulauf 1,
- 1000 g: Wasser
vorgelegt. Die Polymerisationsvorschrift sowie die Starterzuläufe 1 und 2 entsprechen denen von Beispiel 1. Erhalten wurde eine weiße, sedimentationsstabile Dispersion mit einem K-Wert von 61, einem Feststoffgehalt von 20,3 Gew.-% und einem Restmonomergehalt von 200 ppm N-Vinylpyrrolidon. Eine eingedampfte Probe war in Ethanol klar löslich.
- Monomerzulauf:: 190 g Vinylacetat,
45 g N-Vinylpyrrolidon,
2 g Allylalkohol.

Starterzuläufe 1 und 2 entsprechen denen von Beispiel 1.

### Beispiel 6

Dispersion aus 60 Gew.-% N-Vinylpyrrolidon und 40 Gew.-% tertiär-Butylacrylat (anionisches Produkt).

In einem Reaktor mit Rührer, Rückflußkühler, Gaseinlaß und drei getrennten Zuläufen wurde eine Mischung aus
- 50 g: N-Vinylpyrrolidon,
- 0,6 g: 0,01 gew.-%ige Cu^{II}Cl₂-Lösung,
- 5 g: Starterzulauf 1,
- 1000 g: Wasser
vorgelegt. Unter Stickstoffatmosphäre erwärmet man auf 70 °C und gab die Monomerzuläufe 1 und 2 und der Starterzulauf gleichzeitig und mit konstanter Geschwindigkeit über einen Zeitraum von 8 Stunden zu. Die Temperatur wurde danach weitere 6 Stunden bei 75 °C gehalten. Erhalten wurde eine weiße, sedimentationsstabile Dispersion mit einem K-Wert von 78, einem Feststoffgehalt von 19,4 Gew.-% und einem Restmonomergehalt von 50 ppm N-Vinylpyrrolidon. Eine eingedampfte Probe war in Ethanol klar löslich.
- Monomerzulauf 1:: 130 g N-Vinylpyrrolidon,
120 g tertiär-Butylacrylat.
- Monomerzulauf 2:: 3,0 g Acrylsäure mit Natriumhydroxid neutralisiert,
50 g Wasser
- Starterzulauf:: Lösung von 10 g Wasserstoffperoxidlösung (30 Gew.-%) in 50 g Wasser.

### Beispiel 7

Dispersion aus 60 Gew.-% N-Vinylpyrrolidon und 40 Gew.-% tertiär-Butylacrylat (anionisches Produkt).

Die Polymerisationsvorschrift und die Monomerenzuläufe entsprechen denen von Beispiel 6, der Starterzulauf enthielt 20 g Wasserstoffperoxidlösung (30 Gew.-%), gelöst in 50 g Wasser. Erhalten wurde eine weiße, sedimentationsstabile Dispersion mit einem K-Wert von 62, einem Feststoffgehalt von 20,0 Gew.-% und einem Restmonomergehalt von < 50 ppm N-Vinylpyrrolidon. Eine eingedampfte Probe war in Ethanol klar löslich.

### Beispiel 8

Dispersion aus 60 Gew.-% N-Vinylpyrrolidon und 40 Gew.-% tertiär-Butylacrylat (geregelte Fahrweise).

In einem Reaktor mit Rührer, Rückflußkühler, Gaseinlaß und zwei getrennten Zuläufen wurde eine Mischung aus
- 50 g: N-Vinylpyrrolidon,
- 5 g: Starterzulauf 1,
- 1000 g: Wasser
vorgelegt. Die Polymerisationsvorschrift entspricht der von Beispiel 1. Erhalten wurde eine weiße, sedimentationsstabile Dispersion mit einem K-Wert von 79, einem Feststoffgehalt von 19,9 Gew.-% und einem Restmonomergehalt von 70 ppm N-Vinylpyrrolidon. Eine eingedampfte Probe war in Ethanol klar löslich.
- Monomerzulauf:: 130 g N-Vinylpyrrolidon,
120 g tertiär-Butylacrylat,
1 g Mercaptoethanol.
- Starterzulauf 1:: Lösung von 1 g 2,2'-Azobis(2-amidinopropan)dihydrochlorid und 3,0 g Acrylsäure mit Natriumhydroxid neutralisiert in 100 g Wasser.
- Starterzulauf 2:: Lösung von 1 g 2,2'-Azobis(2-amidinopropan)dihydrochlorid in 100 g Wasser.

### Beispiel 9

Dispersion aus 60 Gew.-% N-Vinylpyrrolidon und 40 Gew.-% tertiär-Butylacrylat (geregelte Fahrweise).

Die Polymerisationsvorschrift, die Monomerenzuläufe und der Starterzulauf entsprechen denen von Beispiel 8, wobei der Monomerzulauf 9 g Mercaptoethanol enthält. Erhalten wurde eine weiße, sedimentationsstabile Dispersion mit einem K-Wert von 50, einem Feststoffgehalt von 19,0 Gew.-% und einem Restmonomergehalt von <50 ppm N-Vinylpyrrolidon. Eine eingedampfte Probe war in Ethanol klar löslich.

### Beispiel 10

Dispersion aus 60 Gew.-% N-Vinylpyrrolidon und 40 Gew.-% tertiär-Butylacrylat (kationisches Produkt).

In einem Reaktor mit Rührer, Rückflußkühler, Gaseinlaß und zwei getrennten Zulaufen wurde eine Mischung aus
- 50 g: N-Vinylpyrrolidon,
- 5 g: Starterzulauf 1,
- 1000 g: Wasser
vorgelegt. Die Polymerisationsvorschrift entspricht der von Beispiel 1. Erhalten wurde eine weiße, sedimentationsstabile Dispersion mit einem K-Wert von 38, einem Feststoffgehalt von 20,3 Gew.-% und einem Restmonomergehalt von 300 ppm N-Vinylpyrrolidon. Eine eingedampfte Probe war in Ethanol klar löslich.
- Monomerzulauf:: 121 g N-Vinylpyrrolidon,
120 g tertiär-Butylacrylat.
- Starterzulauf 1:: Lösung von 1 g 2,2'-Azobis(2-amidinopropan)dihydrochlorid und 9,0 g 3-Methyl-1-vinyl-imidazoliummethylsulfat methosulfat als neutralisierte Lösung in 100 g Wasser.
- Starterzulauf 2:: Lösung von 1 g 2,2'-Azobis(2-amidinopropan)dihydrochlorid in 100 g Wasser.

### Beispiel 11

Dispersion aus 60 Gew.-% N-Vinylformamid und 40 Gew.-% tertiär-Butylacrylat (anionisches Produkt).

In einem Reaktor mit Rührer, Rückflußkühler, Gaseinlaß und zwei getrennten Zuläufen wurde eine Mischung aus
- 50 g: N-Vinylformamid,
- 5 g: Starterzulauf 1,
- 1000 g: Wasser
vorgelegt. Die Polymerisationsvorschrift entspricht der von Beispiel 1. Erhalten wurde eine weiße, sedimentationsstabile Dispersion mit einem K-Wert von 56, einem Feststoffgehalt von 19,4 Gew.-% und einem Restmonomergehalt von 350 ppm N-Vinylpyrrolidon. Eine eingedampfte Probe war in Ethanol klar löslich.
- Monomerzulauf:: 130 g N-Vinylformamid,
120 g tertiär-Butylacrylat.
- Starterzulauf 1:: Lösung von 1 g 2,2'-Azobis(2-amidinopropan)dihydrochlorid und 3,0 g Acrylsäure mit Natriumhydroxid neutralisiert in 100 g Wasser.
- Starterzulauf 2:: Lösung von 1 g 2,2'-Azobis(2-amidinopropan)dihydrochlorid in 100 g Wasser.

### Beispiel 12

Dispersion aus 40 Gew.-% N-Vinylpyrrolidon und 60 Gew.-% tertiär-Butylmethacrylat (emulgatorhaltige Dispersion).

In einem Reaktor mit Rührer, Rückflußkühler, Gaseinlaß und zwei getrennten Zuläufen wurde eine Mischung aus
- 10 g: Texapon® NSO (ethoxyliertes Natriumlaurylsulfat)
- 2,2 g: Natriumperoxodisulfat, 7%ige Lösung in Wasser,
- 0,8 g: Ammoniumhydrogencarbonat,
- 20 g: Monomerzulauf,
- 750 g: Wasser
vorgelegt. Die Vorlage wurde mit Stickstoff gespült und auf 80°C Innentemperatur erwärmt. Anschließend wurden unter Aufrechterhaltung der Temperatur der Monomerzulauf und der Starterzulauf 1 gleichzeitig und mit konstanter Geschwindigkeit über einen Zeitraum von 2 Stunden zugegeben. Die Temperatur wurde weitere 2 Stunden bei 80 °C gehalten. Danach wurde Starterzulauf 2 hinzugegeben und eine weitere Stunde bei 80 °C gehalten, nach Zugabe von Starterzulauf 3 wurde die Temperatur weitere 3 Stunden bei 80°C gehalten.

Man erhielt eine weiße, sedimentationsstabile Dispersion mit einem K-Wert von 48, einem Feststoffgehalt von 28,8 Gew.-%, einer Lichtdurchlässigkeit von 70,5 %, einem pH-Wert von 8,2 und einem Restmonomergehalt von 1500 ppm N-Vinylpyrrolidon. Eine eingedampfte Probe war in Ethanol klar löslich.
- Monomerzulauf:: 180 g tertiär-Butylmethacrylat,
120 g N-Vinylpyrrolidon,
0,6 g Thioglykolsäure-2-ethylhexylester (EHTG).
- Starterzulauf 1:: 9 g Natriumperoxodisulfat, 7%ige Lösung in Wasser.
- Starterzulauf 2:: 3,0 g Wasserstoffperoxid (30%ige Lösung), 0,5 g Cu^{II}Cl₂ (0,01%ige Lösung in Wasser).
- Starterzulauf 3:: 1,5 g Wasserstoffperoxid (30%ige Lösung).

### Beispiel 13 bis 20 (emulgatorhaltige Dispersionen)

Die Polymerisation wurde analog Beispiel 12 durchgeführt. Die verwendeten Einsatzmengen an Monomeren sowie die Charakterisierung der Monomere sind in Tabelle 1 zusammengefasst. Für die Monomere wurden folgende Abkürzungen verwendet:
- VP: = N-Vinylpyrrolidon
- VC: = N-Vinylcaprolactam
- t-BuMA: = tert.-Butylmethacrylat
- i-BuMA: = Isobutylmethacrylat
- MMA: = Methylmethacrylat

**Tabelle 1**

| Bsp. | Monomere | | Regler (EHTG) ¹⁾ [g] | K-Wert²⁾ | LD³⁾ [%] | FG⁴⁾ [Gew.-%] | pH | Restmonomere [ppm] |
|---|---|---|---|---|---|---|---|---|
| | A [g] | B [g] | | | | | | |
| 13 | VP 120 | t-BuMA 180 | 1,1 | 41 | 63,5 | 30,6 | 7,0 | VP 2000 |
| 14 | VP 60 | t-BuMa 240 | 0,6 | 45 | 66,5 | 32,1 | 7,6 | VP 2000 |
| 15 | VP 60 | MMA 240 | 0,6 | 47 | 82,0 | 33,1 | 7,8 | VP 500 |
| 16 | VC 90 | t-BuMA 210 | 0,6 | 54 | 77,0 | 28,7 | -⁵⁾ | VC 200 |
| 17 | VC 90 | t-BuMA 210 | 1,1 | 38 | 73,5 | 28,6 | - | VC 500 |
| 18 | VC 120 | t-BuMA 180 | 1,1 | 44 | 93,0 | 28,3 | - | VC 60 |
| 19 | VC 90 | i-BuMA 210 | 1,1 | 43 | 67,5 | 35,2 | - | VC 100 |
| 20 | VC 60 | MMA 240 | 1,1 | 47 | 76,0 | 28,6 | - | VC 300 |

| | | | | | | | | |
|---|---|---|---|---|---|---|---|---|
| ¹⁾ EHTG: 2-Ethylhexylthioglykolat | | | | | | | | |
| ²⁾ K-Wert nach Fikentscher (s. o.) | | | | | | | | |
| ³) LD: Lichtdurchlässigkeit einer 1 gew.-%igen Dispersion (s. o.) | | | | | | | | |
| ⁴⁾ FG: Feststoffgehalt | | | | | | | | |
| ⁵⁾ Wert wurde nicht bestimmt | | | | | | | | |

### III. Verwendung der erfindungsgemäßen Polymerisate als Filmbildner in Haarsprays

### Beispiel 21

Die Polymerisatdispersionen der Beispiele 5, 9, 10 und 13 wurden in bekannter Weise durch Sprühtrocknung in ein Polymerisatpulver überführt.

3 g Copolymer wurden in 62 g Ethanol und 35 g Dimethoxyethan gelöst. Die so erhaltenen Haarsprayformulierungen wurden an Modellköpfen getestet und zeigen ausgezeichnete haarkosmetische Eigenschaften. Bereits ohne weitere Zusätze weisen sie eine exzellente haarverfestigende Wirkung auf. Zur weiteren Optimierung der kosmetischen Eigenschaften können die dem Fachmann bekannten Inhaltsstoffe den Formulierungen zugesetzt werden.

### IV. Verwendung der erfindungsgemäßen Polymerisate als Coating-Retardierungsmittel

Es wurde zunächst eine Pigmentdispersion
- 0,5: Gew.-Teilen Titandioxidpulver
- 4,0: Gew.-Teilen Talkum
- 0,5: Gew.-Teilen Lebensmittelfarbstoff (Sicovit® rot 30) und
- 7,0: Gew.-Teilen Wasser
bereitet und in einer Korundscheibenmühle homogenisiert. Anschließend gab man 58 Gew.-Teile Wasser und 30 Gew.-Teile Dispersion aus Beispiel 1 zu. Die erhaltenen Dispersion hatte einen Feststoffgehalt von 11 Gew.-%.

1745 g dieser Dispersion wurden in einem Aeromatic Strea 1 (Fa. Aeromatic) auf 500 g Theophyllin-Pellets (0,8 bis 1,3 mm; Spherofillin® der Fa. Knoll AG) im Wirbelschichtverfahren aufgesprüht. Die Zulufttemperatur lag bei 45 °C, die Ablufttemperatur bei 30 °C. Die Zuluftmenge lag bei 100 - 130 m³/h. Das Aufsprühen erfolgte kontinuierlich innerhalb 205 min mit einer Aufsprühgeschwindigkeit von 8,5 ml/min und einem Sprühdruck von 0,8 bar. Anschließend trocknete man 5 min im Warmluftstrom (60 °C).

Zur Bestimmung der Freisetzung wurden die beschichteten Pellets in Kapseln (entsprechend 300 mg Theophyllin pro Kapsel) gefüllt und in jeweils 900 ml künstlicher Magensaft (0,1 N Salzäure) eingebracht. Die Freisetzung erfolgte in einer "Paddle-Apparatur" (Fa. Pharmatest) bei 37 °C und 50 U/min. Nach 2 h wurde durch Zugabe eines Phosphatpufferkonzentrats ein pH-Wert von 6,8 eingestellt.

Folgende Freisetzungswerte wurden beobachtet:
- 2 h -: 30,5 %
- 4 h -: 55,6 %
- 6 h -: 80,3 %
- 8 h -: 100,0 %

Als Freisetzungswerte sind die prozentual freigesetzten Theophyllinmengen zu verstehen, wie sie UV-photometrisch bestimmt wurden.

## Patentansprüche

1. Verfahren zur Herstellung wässriger Copolymerisatdispersion durch radikalische, wässrige Emulsionspolymerisation ethylenisch ungesättigter Monomere, umfassend
i) 10 bis 70 Gew.-% wenigstens eines nichtionischen Monomers A mit einer Wasserlöslichkeit oberhalb 60 g/l bei 25 °C, das eine N-Vinylgruppe aufweist,
ii) 30 bis 90 Gew.-% wenigstens eines monoethylenisch ungesättigten, hydrophoben Monomers B mit einer Wasserlöslichkeit unterhalb 60 g/l bei 25 °C sowie gegebenenfalls
iii)0 bis 5 Gew.-% wenigstens eines monoethylenisch ungesättigten Monomers C, das wenigstens eine ionische und/oder ionisierbare funktionelle Gruppe aufweist,
iv) 0 bis 10 Gew.-% wenigstens eines Monomers D, das wenigstens 2 ethylenisch ungesättigte Bindungen aufweist,
v) 0 bis 20 Gew.-% wenigstens eines weiteren, von den Monomeren A und C verschiedenen monoethylenisch ungesättigten Monomers E mit einer Wasserlöslichkeit >60 g/l bei 25 °C,
dadurch gekennzeichnet, dass man einen in Wasser löslichen Polymerisationsinitiator verwendet.

2. Verfahren nach Anspruch 2, dadurch gekennzeichnet, dass der Polymerisationsinitiator ausgewählt ist unter wasserlöslichen Azoverbindungen, Alkali- oder Ammoniumsalzen der Peroxodischwefelsäure und Wasserstoffperoxid.

3. Verfahren nach einem der vorhergehenden Ansprüche, dadurch gekennzeichnet, dass man wenigstens 70 % des Initiators als wässrige oder wässrig-alkoholische Lösung kontinuierlich der Polymerisationsreaktion zuführt.

4. Verfahren nach einem der vorhergehenden Ansprüche, dadurch gekennzeichnet, daß man wenigstens 70 Gew.-% der zu polymerisierenden Monomere kontinuierlich der Polymerisationsreaktion zuführt.

5. Verfahren nach einem der vorhergehenden Ansprüche, dadurch gekennzeichnet, dass die Monomere A ausgewählt sind unter N-Vinyllactamen mit 6 bis 8 C-Atomen und acyclischen N-Vinylcarbonsäureamiden mit 3 bis 6 C-Atomen.

6. Verfahren nach einem der vorhergehenden Ansprüche, dadurch gekennzeichnet, dass die Monomere B ausgewählt sind unter Olefinen, vinylaromatischen Verbindungen, den C₁-C₁₀-Alkylestern monoethylenisch ungesättigter C₃-C₆-Monocarbonsäuren, den Di-C₁-C₁₀-alkylestern ethylenisch ungesättigter C₄-C₆-Dicarbonsäuren, den Vinyl- und (Meth)allylestern linearer oder verzweigter, aliphatischer Carbonsäuren mit 2 bis 20 C-Atomen und den Vinyl- und (Meth)allylethern linearer oder verzweigter, aliphatischer Alkohole, mit 2 bis 20 C-Atomen.

7. Verfahren nach einem der vorhergehenden Ansprüche, dadurch gekennzeichnet, daß man die Polymerisation in Abwesenheit eines Emulgators oder Schutzkolloids durchführt.

8. Wässrige Copolymerisatdispersion, erhältlich durch ein Verfahren nach einem der vorhergehenden Ansprüche.

9. Wässrige Copolymerisatdispersionen nach Anspruch 8, worin die Polymerisatteilchen einen gewichtsmittleren Durchmesser oberhalb 100 nm aufweisen.

10. Verwendung der wässrigen Copolymerisatdispersionen nach einem der Ansprüche 8 oder 9 sowie der daraus durch Trocknung erhältichen Copolymerisate als Hilfsstoffe für pharmazeutische, kosmetische oder agrochemische Zubereitungen, oder zur Herstellung von Anstrich- und Beschichtungsmitteln, Leimen und Klebstoffen.
